# EUROPEAN PATENT APPLICATION

(11) **EP 1 002 525 A2**
(43) Date of publication of application: **24.05.2000**
(21) Application number: 99122747.1
(22) Date of filing: 16.11.1999
(51) Int. Cl.: A61K 7/48, C11D 3/33, A61K 31/4015, A61P 17/00

(54) **N-acylate derivatives of pyrrolidone carboxylic acid as surfactant**

(30) Priority: 18.11.1998 IT TO980968
(71) Applicant: Zschimmer & Schwarz Italiana S.p.A., 13038 Tricerro (Vercelli) (IT)
(72) Inventor: Guala, Fabrizio, 13039 Trino (Vercelli) (IT); Merlo, Elisabetta, 13039 Trino (Vercelli) (IT)
(74) Representative: Rambelli, Paolo

(57) **Abstract**

The present invention relates to the use of N-acylate derivatives of pyrrolidone carboxylic acid of general formula: 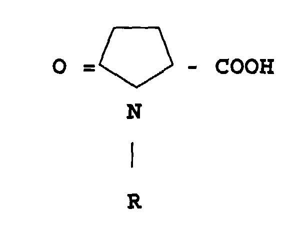 in which R is saturated or unsaturated, linear or branched C₃-C₂₂ acyl and salts thereof as surfactant or hydrating agents in the formulation of a detergent, cosmetic or pharmaceutical composition for topical use or intended for contact with the skin, as well as the use of the said derivatives and their salts for the preparation of pre-formed emulsifying systems as primary emulsifiers.

## Description

The present invention relates to N-acylate derivatives of pyrrolidone carboxylic acid and their salts having surfactant and/or hydrating activity.

Pyrrolidone carboxylic acid (hereinafter called PCA) is a principal component of the Natural Hydrating Factor, that is the set of water soluble substances present in the epidermis indispensable for a correct hydration thereof. It is present almost exclusively in the cortical layer of the epidermis and its preponderant physiological form is the sodium salt which, due to its high hygroscopicity, is able to absorb water up to 60% of its own weight. The other components of the Natural Hydrating Factor are principally sugars, amino acids, urea, and lactates.

PCA, moreover, forms part of a complex series of biological cycles. It is synthesised by the action of the enzymes L-glutamate cyclotransferase, L-glutamine cyclotransferase, L-γ-glutamylcyclotransferase and L-pyrrolidone carboxypeptidase starting from glutamic acid, glutamine, γ-glutamyl amino acids and peptides, respectively, whilst it constitutes in turn the biochemical precursor of proline and hydroxyproline, two extremely abundant amino acids in the collagen. PCA is moreover one of the products of degradation of filaggrin, a protein produced by keratinocytes capable of organising and stabilising the keratin filaments thereby contributing to the correct functionality of the skin.

On the basis of these characteristics, PCA and its sodium salts are widely utilised in cosmetics as hydrating agents.

An ester derivative of PCA is also known in which the carboxylic group has been esterified with dodecanol fatty acid to give lauryl PCA. This molecule is insoluble in water and is utilised above all as an emulsifier.

Muranishi Shozo et al., Pharm Res (1991), 8(5), 649-52 describes the synthesis of the lauroyl derivative of TRH (thyrotropin releasing hormone) obtained by bonding the lauric acid to the nitrogen of the pyroglutamyl group of TRH; this derivative has an increased lipophily and finds application exclusively in the pharmaceutical field as a TRH analogue.

The present invention is based on the discovery of the fact that the amide derivatives of PCA - in which the hydrogen atom bound to the nitrogen of the ring is substituted by an acyl group to give rise to an amide - and their salts have excellent properties as surfactants together with hydrating properties which make their use advantageous in detergent, cosmetic and/or pharmaceutical compositions for topical use or intended to contact the skin, as well as for the preparation of pre-formed emulsifying systems as primary emulsifiers.

Therefore, a subject of the present invention is the use of N-acylate derivatives of PCA of the general formula: 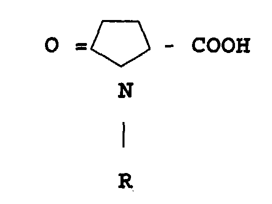 in which R is a saturated or unsaturated, linear or branched C₃-C₂₂ acyl, or salts thereof as surfactant or hydrating agents in the formulation of a detergent, cosmetic or pharmaceutical composition for topical use. The said salt is preferably the salt of an alkali metal such as sodium, potassium or lithium, an alkali earth metal, a transition metal such as chromium, iron, copper, manganese or zinc, ammonium or an amine base such as triethanolamine, monoethanolamine, diethanolamine, monoisopropanolamine, triisopropanolamine, 2-aminobutanol, aminoethylpropandiol, arginine, lysine, ornithine, aminomethylpropanol, aminomethylpropandiol or 2-amino-2-hydroxymethyl-1,3-propandiol. Derivatives are preferred in which R is the acyl of a C₁₂-C₁₈ fatty acid such as lauric, myristic, palmitic, stearic, oleic, linoleic, or linolenic acid.

Also within the scope of the invention is the use of the said N-acylate derivatives of PCA and their salts in emulsions for cosmetic and pharmaceutical use either as emulsifying agents or components of pre-formed emulsifying systems, and as active principles, such as for example hydrating agents.

Another subject of the present invention is a detergent, cosmetic or pharmaceutical composition for topical use comprising at least one derivative of PCA or a salt thereof as previously described and an aqueous medium.

The above derivatives, with the exception of N-lauroylpirrolidone carboxylic acid, are new compounds and constitute a further subject of the invention.

The said derivatives and their salts can be obtained starting from PCA by the Schotten-Baumann reaction already widely described in the prior art (C. Schotten, Ber. 17, 2544 (1884); E. Baumann, Ber. 19, 3218 (1886)).

The acylation of PCA on the nitrogen atom makes it possible to obtain molecules characterised by the presence of a lipophilic group (acyl chain) and a hydrophilic head (carboxylic group) usable as surfactants in both rinseable and leave-on toiletry products and in detergent formulations for personal care.

The discovered efficacy of the derivatives according to the invention as hydrating surfactants leads one to believe that their administration in contact with the skin leads, by the action of enzymatic systems present on the skin and able to hydrolyse the amide bond, to the formation of free fatty acids which can form part of various biological cycles, and of PCA, which has the previously described hydrating properties.

In the detergent, cosmetic or pharmaceutical compositions of the invention the derivative of PCA or its salt can be present as a single or principal surfactant or else in combination with other surfactant agents such as anionic surfactants, for example salts of alkyl sulphates, salts of alkyl ether sulphates, salts of amido/amidoether sulphates, salts of alkyl semisulphosuccinates and of alkylsulphosuccinates, salts of alkylethersemisulphosuccinates and of alkylethersulphosuccinates, salts of acylamidosemisulphosuccinates and of acylamidosulphosuccinates, salts of acylated amino acids such as, for example, salts of acylsarcosinate, salts of peptides and acylated proteins, salts of fatty acids, salts of dodecylbenzensulphonic acid, salts of alkyl/alkylether sulphoacetates, salts of sulphonated and/or sulphated organic molecules such as, for example sulphonated α-olefins, salts of alkyl/alkylether carboxylates, alkylphosphonates, esters of phosphoric acid, salts of acylisethionates; amphoteric surfactants such as, for example, alkyl betaine, alkyl amidopropylbetaine, oxides of amines and amides, hydrolysed proteins, amphocarboxyacetates and amphocarboxydiacetates, amphocarboxypropionates and amphocarboxydipropionates; nonionic surfactants such as, for example, amides, optionally ethoxylated fatty amines, ethoxylated nonylphenols, alkyl polyglucosides, alkyl ethoxyglucosides, esters/ethers of fatty acids with glycerol and/or optionally ethoxylated sugars, optionally ethoxylated/propoxylated esters, sorbitol, glycerine, glycols, optionally ethoxylated/propoxylated fatty alcohols, siliconic molecules; or cationic surfactants containing, for example, at least one quaternary nitrogen atom.

The concentration of the derivative of PCA or its salt in the aqueous compositions can vary within wide limits. A sufficient surfactant activity is obtained even at low concentrations of the order of about 0.1% by weight referred to the total composition: preferred concentrations are of the order of 1-30% by weight, more preferred of 2-10% by weight.

As previously stated, the N-acylate derivatives of PCA and their salts can be utilised as components of pre-formed emulsifying systems. These latter are constituted by a primary hydrophilic emulsifier which orientates the emulsion towards the O/A form and a secondary lipophilic emulsifier (or a mixture of lipophilic emulsifiers) which stabilises the emulsion. By using these pre-formed emulsifying systems it is possible to obtain a greater consistency and stability of emulsions of O/A type.

Such pre-formed emulsifying systems, which constitute a further subject of the invention, comprise an N-acylate derivative of PCA or a salt thereof as primary emulsifier, and a secondary emulsifier which is a long chain linear or branched, saturated or unsaturated C₆-C₃₀ alcohol having one or more hydroxylic functions optionally ethoxylated/propoxylated, possibly in combination with another secondary emulsifier selected from acid, ester and their mixtures in which:
- the acid is a carboxylic acid having one or more carboxylic functions; and
- the ester is obtained by reaction between an acid as previously described and an alcohol as previously described or a short chain linear or branched, saturated or unsaturated C₂-C₆ alcohol having one or more hydroxylic functions optionally ethoxylated/propoxylated, such as, for example, glycerine or propylene glycol.

In a preferred embodiment of the invention the N-acylate derivative of PCA or its salt is present in the pre-formed emulsifying system at a concentration of 1% to 50%, preferably 10% to 20%; the long chain alcohol is present at a concentration of 1% to 90% preferably 20% to 40%; the carboxylic acid is present at a concentration up to 70%, preferably 20% to 30%, and the ester is present at a concentration up to 90%, preferably 30% to 40%. These concentrations are expressed in terms of weight with respect to the dry weight of the pre-formed emulsifying system.

Products in which the derivatives of PCA or its salts previously described can be utilised include for example shampoo, bath foam, shower foam, washing up liquid, liquid and solid soaps, lotions, emulsions of various nature, balsams, products having simultaneous detergent and conditioning effect on the skin and/or hair, oils, emollient and detergent milks, creams for the face and/or the body and/or the hair, detergents for intimate hygiene, brilliantine, solutions for permanent waving or colouring hair, medicated cosmetics, dentifrices and pharmaceutical products.

The formulations containing these ingredients exhibit an optimum hydrating power, have exceptional gentleness, good foaming property and rinseability.

The following examples relating to cosmetic formulations in which the use of the previously-described family of molecules can be found are provided by way of illustration only and are not intended to limit in any way the scope of the present invention.

### GENTLE SYNDET SOAP

| | |
|---|---|
| Sodium cocoyl isethionate | 40% |
| N-Palmitoyl PCA sodium salt | 10% |
| Stearic acid | 34% |
| Soap chip (80/20 tallow/coco) | 10% |
| Perfume, pigments, etc. | 1% |
| Deionised water | 5% |

### DETERGENT FOR INTIMATE HYGIENE

| | |
|---|---|
| Sodium laureth-3,7 sulphate (27% active material) | 30% |
| N-Cocoyl PCA sodium salt | 4% |
| Lauramidopropyl betaine | 5% |
| C₁₂-C₁₄ alcohol 3 OE | 2% |
| Disodium laureth-3 sulphosuccinate (30% active material) | 4% |
| Water, perfume, preservative and dye | to 100% |

### BATH FOAM

| | |
|---|---|
| Sodium laureth-3 sulphate (27% active material) | 25% |
| N-LauroylPCA sodium salt | 3.5% |
| Disodium cocoamphodiacetate (26% active material) | 5% |
| Pearliser | 2.5% |
| NaCl | 1% |
| Citric acid | 0.1% |
| Water, perfume, preservative and dye | to 100% |

### FOAMING BATH OIL

| | |
|---|---|
| Sodium laureth-2 sulphate (27% active material) | 55% |
| N-lauroylPCA sodium salt | 2.5% |
| Cocamidopropyl betaine (30% active material) | 5% |
| Cocamido DEA | 2.5% |
| PEG-7 glyceryl cocoate | 1.5% |
| Lauramidopropylamine oxide (30% active material) | 2.5% |
| PEG-6 Caprylic/capric glycerides | 1.5% |
| Water, perfume, preservative and dye | to 100% |

### SHOWER GEL

| | |
|---|---|
| Sodium laureth-2 sulphate (70% active material) | 28% |
| N-CocoylPCA sodium salt | 3% |
| Disodium cocoamphodiacetate (26% active material) | 4% |
| Pearliser | 2.5% |
| Disodium laureth-3 sulphosuccinate (30% active material) | 4% |
| Lactic acid | to pH 5.5 |
| NaCl | 1% |
| Extract of Aloe | 0.55% |
| Water, perfume, preservative and dye | to 100% |

### GENTLE LIQUID SOAP

| | |
|---|---|
| Sodium laureth-2 sulphate (70% active material) | 20% |
| N-CocoylPCA sodium salt | 2% |
| Lauramido propyl betaine (30% active material) | 8% |
| C₁₂-C₁₄ Alcohol 3 OE | 1% |
| Sodium Lauroyl Glutamate (30% active material) | 1.5% |
| Glycerine | 1% |
| Pearliser | 2.5% |
| Water, perfume, preservative and dye | to 100% |

### DENTRIFICE

| | |
|---|---|
| Calcium carbonate | 48% |
| Carrageenan | 0.94% |
| Glycerine | 22% |
| N-lauroylPCA sodim salt | 1.0% |
| Aromatic oil | 0.8% |
| Sodium saccharin | 0.2% |
| Sodium benzoate | 0.5% |
| Water | to 100% |

### WASHING UP LIQUID - GENTLE ON THE HANDS

| | |
|---|---|
| (22% active material) | |
| Sodium dodecylbenzene- sulphonate (30% active material) | 40% |
| Sodium laureth-3 sulphate (70% active material) | 7% |
| Cocamide DEA | 2.6% |
| N-CocoylPCA sodium salt | 2.5% |
| Water, perfume, preservative and dye | to 100% |

### DETERGENT FOR WOOL

| | |
|---|---|
| Sodium laureth-3 sulphate (70% active material) | 40% |
| N-CocoylPCA sodium salt | 1% |
| Lauramido propyl betaine (30% active material) | 2% |
| Potassium cocoate (35% active material) | 4% |
| C₁₂-C₁₄ alcohol EO/PC adduct | 3% |
| Cocamide DEA | 1% |
| NaCl | 2% |
| Water, perfume, preservative and dye | to 100% |

### SHAMPOO

| | |
|---|---|
| Sodium laureth-3 sulphate (70% active material) | 16% |
| Lauramido propyl betaine (30% active material) | 3% |
| N-lauroylPCA sodium salt | 3% |
| NaCl | 2.4% |
| Lauramido propylamine oxide (30% active material) | 3% |
| Water, perfume preservative and dye | to 100% |
| Lactic acid | to desired pH |

### DAY CREAM

| Phase A: | |
|---|---|
| N-lauroylPCA sodium salt | 1% |
| Glyceryl stearate | 1.8% |
| Stearic acid | 1% |
| Cetearyl alcohol | 2.8% |
| Ascorbyl palmitate | 0.5% |
| Caprylic/capric triglyceride | 9% |
| Cetearyl isononoate | 8% |
| Hexyl laurate | 3% |
| Dimethicone | 0.5% |

| Phase B: | |
|---|---|
| Glycerine | 5% |
| Xanthan gum | 0.25% |
| Water | 67.15% |

Heat phase B to 60°C and homogenise it, then warm phase A to 60°C and add it under agitation to phase B. Homogenise all. Cool under agitation to 45°C. Homogenise for 30 seconds. Continue cooling down to ambient temperature under slow agitation.

## Claims

1. Use of N-acylate derivatives of pyrrolidone carboxylic acid of the general formula: 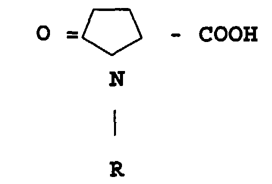 in which R is saturated or unsaturated linear or branched C₃-C₂₂ acyl, and salts thereof as surfactant or hydrating agents in the formulation of a detergent, cosmetic or pharmaceutical composition for topical use.

2. Use according to Claim 1 in which the said salt is an alkali metal, alkaline earth, transition metal, ammonium or amine base salt.

3. Use according to Claim 2 in which the amine base is selected from triethanolamine, monoethanolamine, diethanolamine, monoisopropanolamine, triisopropanolamine, 2-aminobutanol, aminoethylpropandiol, arginine, lysine, ornithine, aminomethylpropanol, aminomethylpropandiol and 2-amino-2-hydroxymethyl-1,3-propandiol.

4. Use according to any of Claims 1 to 3 in which R is the acyl of a C₁₂-C₁₈ fatty acid such as lauric acid, myristic acid, palmitic, stearic, oleic, linoleic or linolenic acid.

5. N-acylate derivatives of pyrrolidone carboxylic acid and its salts as described in any of Claims 1 to 4, having surfactant and/or hydrating activity, excluding N-lauroylpirrolidone carboxylic acid.

6. A detergent, cosmetic or pharmaceutical composition for topical use comprising at least one compound according to one of Claims 1 to 4 and an aqueous medium.

7. A composition according to Claim 6, in which the said compound is present at a concentration of 0.1% to 30% by weight with respect to the total composition.

8. A composition according to Claim 7, in which the said compound is present at a concentration of 2% to 10% by weight with respect to the total composition.

9. A pre-formed emulsifying system comprising at least one compound according to any of Claims 1 to 4 and a secondary emulsifier comprising a linear or branched, saturated or unsaturated long chain C₆-C₃₀ alcohol having one or more hydroxylic functions.

10. A pre-formed emulsifying system according to Claim 9, in which the said hydroxylic functions are ethoxylated and/or propoxylated.

11. A pre-formed emulsifying system according to Claim 9 or Claim 10 which further includes a secondary lipophilic emulsifier selected from a carboxylic acid having one or more hydroxylic functions, an ester obtained by reaction of the said carboxylic acid with the said long chain alcohol or with a linear or branched, saturated or unsaturated short chain C₂-C₆ alcohol having one or more hydroxylic functions, and mixtures thereof.

12. A pre-formed emulsifying system according to Claim 11 in which the said hydroxylic functions are ethoxylated and/or propoxylated.
